## Europäisches Patentamt

## European Patent Office

## Office européen des brevets

(19)

(11) Veröffentlichungsnummer: **0 155 586**
**B1**

(12)

# EUROPÄISCHE PATENTSCHRIFT

(45) Veröffentlichungstag der Patentschrift:
**28.10.87**

(21) Anmeldenummer: **85102446.3**

(22) Anmeldetag: **05.03.85**

(51) Int. Cl.⁴: **C 07 C 79/10,** C 07 C 76/02

(54) **Verfahren zur Herstellung von Dinitrotoluol.**

(30) Priorität: **16.03.84 DE 3409719**

(43) Veröffentlichungstag der Anmeldung:
**25.09.85 Patentblatt 85/39**

(45) Bekanntmachung des Hinweises auf die Patenterteilung:
**28.10.87 Patentblatt 87/44**

(84) Benannte Vertragsstaaten:
**BE DE FR GB IT NL**

(56) Entgegenhaltungen:
**EP - A - 0 022 181**
**EP - A - 0 066 202**

(73) Patentinhaber: **BAYER AG, Konzernverwaltung RP Patentabteilung, D-5090 Leverkusen 1 Bayerwerk (DE)**

(72) Erfinder: **Gerken, Rudolf, Dr., Rather Strasse 79, D-4150 Krefeld 1 (DE)**
Erfinder: **Lailach, Günther, Dr., Bodelschwinghstrasse 23, D-4150 Krefeld 1 (DE)**
Erfinder: **Becher, Dieter, Dr., Moltkestrasse 8, D-4047 Dormagen (DE)**
Erfinder: **Witt, Harro, Dr., Möhlenbarg 2, D-2224 Kuden (DE)**

ACTORUM AG

**Beschreibung**

Die vorliegende Erfindung betrifft ein Verfahren zur Herstellung von Dinitrotoluol durch zweistufige Umsetzung von Toluol mit Salpetersäure in Gegenwart von Schwefelsäure, wobei in der ersten Stufe Toluol zu Mononitrotoluol nitriert wird unter Verwendung der Absäure aus der zweten Stufe, in der das Mononitrotoluol zu Dinitrotoluol nitriert wird unter Verwendung der im Vakuum aufkonzentrierten Absäure aus der ersten Stufe.

Die Entsorgung oder Wiederverwendung von bei der Herstellung von Dinitrotoluol anfallender gebrauchter Schwefelsäure stellt insofern ein Problem dar, als die Schwefelsäure einen wesentlichen Anteil der Herstellkosten verursacht. Das führte einerseits zu Versuchen, den Einsatz von Schwefelsäure überhaupt zu vermeiden (Kirk-Othmer, Encycl. Chem. Techn. 3. Aufl. 1981, Bd. 15, S. 928-929), andererseits zu Bemühungen, die Schwefelsäure durch mehr oder weniger aufwendige Reinigungsverfahren (US-PS 4 257 986) für den Einsatz in der Düngemittelindustrie oder für die Rezirkulierung nach vorheriger Eindampfung brauchbar zu machen.

Das am meisten verbreitete Verfahren zur Eindampfung von gebrauchter Schwefelsäure ist das Pauling-Verfahren [Bodenbrenner, von Plessen, Vollmüller, Dechema-Monogr. 86 (1980), 197], bei dem eine relativ reine 96%ige Schwefelsäure zurückgewonnen werden kann.

Die Nachteile dieses Verfahrens liegen in den hohen spezifischen Investitions- und Betriebskosten; durch oxidative Zerstörung eines Teils der organischen Verbindungen entstehen ausserdem $SO_2$ und $NO_x$-Verbindungen und -Gase.

Eine weitgehende Entfernung bzw. Rückgewinnung der organischen Verbindungen aus der schwefelsäurehaltigen Absäure kann gemäss der US-PS 3 856 673 durch Strippen mit Wasserdampf bei Temperaturen von 130 bis 230°C erfolgen.

Die an sich bekannten Verfahren zur Vakuumeindampfung von Schwefelsäure (Winnacker . Küchler, Chem. Technol., Bd. 2, Anorg. Technol. I. 4, Aufl., 1982, S. 70-72) zeigen bei der Eindampfung von unbehandelter gebrauchter Schwefelsäure (im folgenden als «Absäure» bezeichnet) aus der Dinitrotoluol-Herstellung eine Reihe von Problemen, die ihre Anwendung verhinderten. Die Absäuren enthalten bei optimaler Führung des Toluolnitrierungsprozesses neben Wasser und Metallsulfaten vor allem Nitrosylschwefelsäure, Dinitrotoluole (DNT), Mononitrotoluole (MNT) und Salpetersäure.

DNT und MNT sind wasserdampfflüchtig und werden zusammen mit dem Wasser weitgehend ausgedampft. Bei den unter Vakuum notwendigen tiefen Kondensationstemperaturen kristallisiert DNT aus und führt zu Verstopfungen im Kondensationssystem. Die direkte Brüdenkondensation in Einspritzkondensatoren mit frischem Kühlwasser ist nicht wirtschaftlich durchführbar, weil dabei zu grosse Mengen belasteten Abwassers anfallen und das in der Absäure enthaltene MNT und DNT verloren geht. Wird aber ein indirekt gekühlter Kreislauf aus Brüdenkondensat aufrechterhalten, so treten durch festes DNT ähnliche Probleme auf wie bei der Brüdenkondensation an gekühlten Wärmetauscherflächen.

Die Vermeidung dieser Probleme erwies sich als kaum realisierbar. Die extraktive Entfernung der Organika vor der Eindampfung der Säure führt zu völlig unbefriedigenden Ergebnissen. Durch aufwendiges Strippen mit Dampf kann zwar der überwiegende Teil der organischen Verbindungen entfernt werden, doch verbleiben insbesondere erhebliche Mengen 2,4-Dinitrotoluol in der Absäure.

In der EP-A 66 202 ist ein Verfahren beschrieben, wonach 2,4-Dinitrotoluol aus der Absäure vor deren Rekonzentrierung und Wiederverwendung bei der Dinitrierung durch Abkühlen der Absäure als nahezu reine Substanz abgeschieden wird. Dadurch verringert sich der DNT-Gehalt der Absäure auf ca. 50% des vorherigen Gehaltes, entsprechend den dortigen Angaben auf 1 - 4 Gew.-%. Erfahrungsgemäss treten aber bei der an sich wirtschaftlich vorteilhafteren Vakuumeindampfung von Absäure aus der Toluolnitrierung bereits bei einem DNT-Gehalt von 0,5 Gew.-% oder weniger Probleme durch Verstopfungen im Bereich der Brüdenkondensation infolge Abscheidens von festem DNT auf. Auch das in der EP-A 22 181 generell für die Rekonzentrierung organisch belasteter Schwefelsäuren vorgeschlagene Verfahren ist im Fall DNT-haltiger Absäuren nicht anwendbar. Die in der EP-A 66 202 beschriebene Rekonzentrierung der stark DNT-haltigen Absäure kann nur eine Eindampfung bei hohen Temperaturen ohne Vakuum sein.

Das Ziel der vorliegenden Erfindung ist es, die Rezirkulierung der bei der Herstellung von Nitrotoluolen anfallenden gebrauchten Schwefelsäure nach einem neuen Verfahren durchzuführen, das gegenüber den bekannten Verfahren zu einer ökonomischen und ökologischen Verbesserung führt. Ein weiteres Ziel ist es, die organischen Verbindungen dabei weitestgehend in den Nitrierungsprozess zurückzuführen und damit die Wirtschaftlichkeit der Herstellung von Dinitrotoluolen (DNT) weiter zu verbessern.

Überraschend wurde nun gefunden, dass alle beschriebenen Probleme vermieden werden können, wenn in die überhitzten Brüden MNT eingesprüht wird, bevor diese in den indirekt gekühlten Brüdenkondensator gelangen. Erfindungsgemäss wird auf eine Entfernung der organischen Verbindungen vor der Eindampfung der Absäure verzichtet, wodurch das in die Brüden eingespritzte MNT dabei relativ stark mit Toluol oder DNT verunreinigt sein kann.

Gegenstand der vorliegenden Erfindung ist somit ein Verfahren zur Herstellung von Dinitrotoluol durch zweistufige Umsetzung von Toluol mit Salpetersäure in Gegenwart von Schwefelsäure, wobei in der ersten Stufe Toluol zu Mononitrotoluol nitriert wird unter Verwendung von Absäure aus der zweiten Stufe, in der das Mononitrotoluol zu Dinitrotoluol nitriert wird unter Verwendung von aufkonzentrierter Absäure aus der ersten Stufe, welches dadurch gekennzeichnet ist, dass die Absäure in indirekt beheizten Eindampfern unter Vakuum aufkonzentriert wird und Mononitrotoluol in die überhitzten Brüden des Eindampfers gegeben wird.

Besonders vorteilhaft wird das Mononitrotoluol in Form von mononitrotoluolhaltigen Gemischen, beborzugt als Teil der organischen Phase, die nach der Toluolmononitrierung abgetrennt wird, zugegeben.

Die Zugabe kann auch in die Absäure selbst erfolgen, jedoch ist aus energetischen Gründen das Einsprühen in die überhitzten Brüden vorzuziehen. Vorteilhaft wird gleichzeitig Wasser oder bevorzugt ein Teil der wässrigen Phase des Brüdenkondensates in die Brüden eingedüst, damit die Brüden als Sattdampf in den Kondensator gelangen. Dies erlaubt, die Kühlflächen relativ klein zu halten.

Die anfallende Absäure hat im allgemeinen eine $H_2SO_4$-Konzentration von 65 bis 83% und weist darüber hinaus vor allem Toluol, MNT, DNT und $NO_x$ auf.

In einer besonders bevorzugten Ausführungsform des erfindungsgemässen Verfahrens wird die Absäure auf einen Schwefelsäuregehalt von 88 bis 94% $H_2SO_4$ eingedampft und bei der Dinitrotoluolherstellung und danach bei der Mononitrotoluolherstellung eingesetzt.

Die Eindampfung der Absäure geschieht erfindungsgemäss in Horizontal-, Fallfilm- oder Umlaufverdampfern.

Im Gegensatz zu den mit Erdgas oder Heizöl befeuerten Pauling-Kesseln kann bei den bevorzugten Eindampfverfahren Dampf als Energieträger verwendet werden. Durch Wärmetausch zwischen eingespeister Absäure und aufkonzentrierter Säure kann der Energieverbrauch gegenüber dem Pauling-Verfahren insgesamt auf ca. 60% gesenkt werden.

Es ist als vorteilhaft anzusehen, den Eindampfprozess in mehrere Stufen zu zerlegen, die jeweils bei optimalen Bedingungen betrieben werden können. Bis zu Konzentrationen von 94% $H_2SO_4$ bietet daher der Horizontalverdampfer Vorteile. Die wässrige Phase des Brüdenkondensats enthält nur sehr geringe Mengen Schwefelsäure.

Durch ein Optimieren der Produktströme können weitere energetische Vortele erzielt werden. So ist es besonders vorteilhaft, einen Teil der nach der Dinitrotoluolherstellung abgetrennten Absäure bei der Mononitrotoluolherstellung einzusetzen und danach zusammen mit dem anderen Teil der Absäure aus der Dinitrotoluolherstellung auf einen Schwefelsäuregehalt von 88 bis 94% einzudampfen.

Eine weitere Ausführungsform des erfindungsgemässen Verfahrens besteht darin, dass die 88- bis 94%ige Schwefelsäure bei der Dinitrotoluolherstellung zusammen mit 98- bis 100%iger $HNO_3$ und anschliessend bei der Mononitrotoluolherstellung zusammen mit 63- bis 70%iger $HNO_3$ eingesetzt wird.

Die erfindungsgemäss eingedampfte Säure kann schliesslich noch einer Hochkonzentrierung unterzogen werden. Dieser Prozess kann in Umlaufverdampfern, Pauling-Kesseln oder nach dem BAYER-Bertrams-Verfahren (DE-OS 3 018 665) erfolgen.

Eine Ausführungsform des erfindungsgemässen Verfahrens besteht also darin, dass die Absäure auf einen Schwefelsäuregehalt von 88 bis 94% eingedampft wird und anschliessend in einer Hochkonzentrierungsstufe auf 94 bis 97% $H_2SO_4$ eingedampft wird, bevor sie wieder bei der Dinitrotoluolherstellung eingesetzt wird.

Erfindungsgemäss wird die gesamte bei der Absäureeindampfung anfallende organische Phase in den Nitrierungsprozess zurückgeführt, wodurch die DNT-Ausbeute merklich erhöht wird. Der Verbund von Schwefelsäureeindampfung und DNT-Herstellung stellt demzufolge einen wesentlichen Vorteil der Erfindung dar.

Zur Entfernung von $NO_x$ kann die Absäure vor der Eindampfung mit $SO_2$, Amidosulfonsäure, Harnstoff oder Ammoniumsulfat behandelt werden. Sie kann aber auch mit Dampf gestrippt werden, wobei die Nitrose nach Kondensation des Wasserdampfes und der Wasserdampf-flüchtigen Verbindungen in Natronlauge unter Bildung von Natriumnitrit absorbiert und einer Weiterverwendung zugeführt werden kann. Alternativ können die nitrosen Gase in einer reduzierenden Flamme zerstört werden.

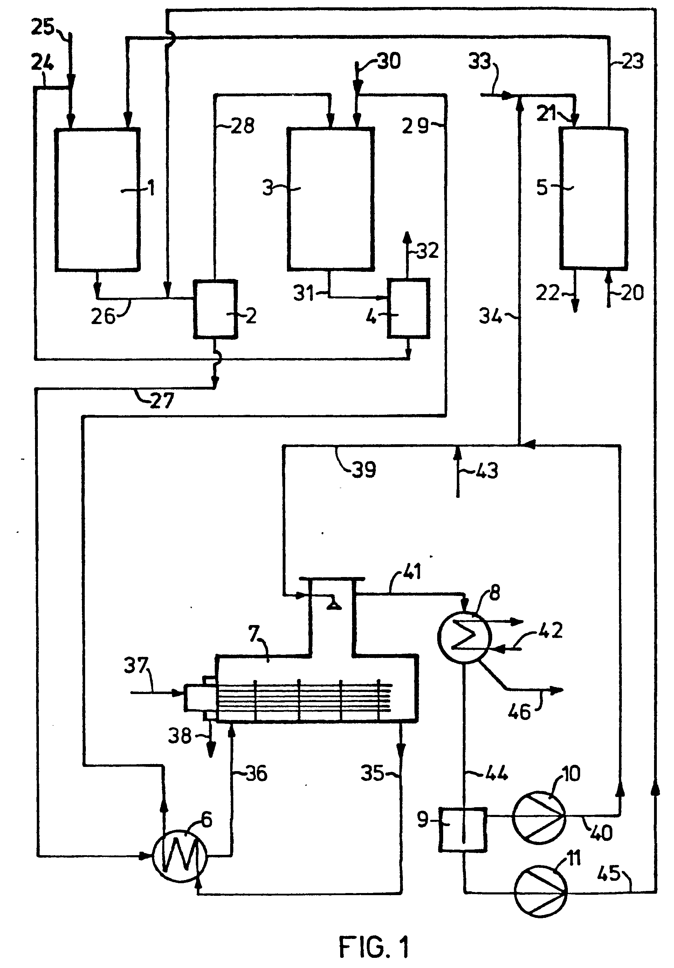

Eine bevorzugte Ausführungsform des erfindungsgemässen Verfahrens wird anhand von Fig. 1 ausführlicher dargestellt. Die Konzentrationsangaben sind dabei nur beispielhaft zu sehen, sie stellen keine Einschränkung der Erfindung auf die genannten Werte dar. Vielmehr ist die Erfindung in einem breiteren Rahmen anwendbar.

Im folgenden wird die Herstellung von Dinitrotoluol (DNT) unter Einsatz von 88- bis 94%iger Schwefelsäure und 99%iger Salpetersäure und die Eindampfung der Absäure von 70 bis 82% auf eine Schwefelsäurekonzentration von 88 bis 94% in einem Horizontalverdampfer dargestellt.

Toluol (20) wird in die Extraktionskolonne (5) eingespeist, in der MNT und DNT aus dem Abwasser (21) extrahiert werden. Das aus der Kolonne (5) austretende MNT- und DNT-haltige Toluol (23) wird in die Mononitrierung (1) eingespeist. Gleichzeitig werden die einen Schwefelsäuregehalt von 80 bis 86% aufweisende Absäure (24), die im Separator (4) vom DNT abgetrennt wurde, und Salpetersäure (25) in die Mononitrierung (1) eingespeist. Das aus der Mononitrierung ausgespeiste Gemisch (26) wird im Separator (2) getrennt. Die 70- bis 82%ige Absäure (27) fliesst zur Säureeindampfung. Die organische Phase (28), die vorwiegend aus MNT besteht, wird in die Dinitrierung (3) eingespeist, zusammen mit 88- bis 94%iger Schwefelsäure (29) aus der Säureeindampfung und frischer ca. 99%iger Salpetersäure (30). Das aus der Dinitrierung (3) ausgespeiste Gemisch (31) wird im Separator (4) getrennt. Die Absäure (24) gelangt zur Mononitrierung (1).

Das Roh-DNT (32) wird auf übliche Weise säurefrei gewaschen. Das dabei anfallende DNT- und MNT-haltige Wasser (33) wird zusammen mit der wässrigen Phase des Brüdenkondensats aus der Absäureeindampfung (34) der Extraktionskolonne (5) zugeführt und durch Extraktion mit Toluol (20) von Nitroverbindungen befreit, bevor es als organisch belastetes Abwasser (22) einer Abwasserbehandlung zugeführt wird.

Die 70- bis 82%ige Absäure (27) aus der Mononitrierung (1), die im Separator (2) vom MNT (28) abgetrennt wurde, wird im Wärmetauscher (6) im Gegenstrom durch die aus dem Horizontalverdampfer (7) ausgespeiste 88- bis 94%ige Schwefelsäure (35) auf 100 bis 130°C aufgeheizt (36) und in den Verdampfer (7), gegebenenfalls über einen vorgeschalteten Flashverdampfer, eingespeist.

Der Wärmetauscher des Horizontalverdampfers (7) besteht aus einem Bündel Tantalrohre, das mit Dampf (37) von 170 bis 210°C, vorzugsweise 170 bis 197°C, beheizt wird. Das Kondensat (38) kann vorteilhaft bei der Dampferzeugung eingesetzt werden.

Die Verdampfung erfolgt bei einem Druck von 20 bis 150 mbar, vorzugsweise 40 bis 100 mbar. Die mit 170 bis 195°C, vorzugsweise 170 bis 185°C, aus dem Verdampfer (7) ausgespeiste 88- bis 94%-ige Schwefelsäure (35) wird im Wärmetauscher (6) auf ca. 60°C gekühlt. Vor der Einspeisung der Säure (27) in die Dinitrierung (3) empfiehlt sich eine weitere Kühlung mit Wasser auf ca. 40°C.

Die überhitzten Brüden werden durch Einspritzen von Wasser oder bevorzugt eines Teils (39) der wässrigen Phase (40) des Brüdenkondensats auf Sattdampftemperatur gekühlt. Um die Erstarrung organischer Verbindungen im Brüdenleitungs- und in dem mit Wasser (42) gekühlten Kondensationssystem (8) zu vermeiden, wird MNT (43), bevorzugt die organische Phase (28) aus der Mononitrierung (1), separat oder zusammen mit der wässrigen Phase in die überhitzten Brüden eingespeist. Die erforderliche MNT-Menge ist um so grösser, je geringer die Kondensationstemperatur und je kleiner das MNT zu DNT-Verhältnis in den Brüden ist.

Das Verhältnis von MNT zu DNT in der organischen Phase des Brüdenkondensats soll erfindungsgemäss mindestens 2:1 betragen und in jedem Fall kleiner als 10:1 sein. Bevorzugt wird ein Verhältnis von 4:1 bis 7:1. In der Praxis wird das MNT oder MNT-haltige Gemisch in solchen Mengen zugegeben, dass die Abscheidung fester Dinitrotoluole in der Anlage mit Sicherheit vermieden wird.

Das Brüdenkondensat (44) fliesst in eine Vorlage (9) ab, aus der die organische Phase (45) mittels Pumpe (11) abgezogen und zur Nitrierung gefördert wird und die wässrige Phase (40) mittels Pumpe (10) abgezogen und zur Abwasserextraktion (5) gefördert wird. Die organische Phase des Brüdenkondensats (45) wird vorzugsweise unmittelbar nach der Mononitrierung (1) in das Nitrierungssystem eingespeist. Die nichtkondensierbaren Anteile (46) der Brüden (41) werden nach der Brüdenkondensation zur Vakuumpumpe abgezogen. Als Vakuumpumpe wird eine Flüssigkeitsringpumpe bevorzugt, die mit Wasser oder Schwefelsäure, bevorzugt der eingedampften Schwefelsäure vor deren Rückführung zur Dinitrierung (3), als Sperrflüssigkeit betrieben wird.

In einer anderen Ausführung des erfindungsgemässen Verfahrens wird ein Teilstrom der hierbei 83- bis 86%igen Schwefelsäure (24) direkt zum Wärmetauscher (6) geleitet und nur soviel Schwefelsäure zur Mononitrierung (1) geleitet, dass die aus dieser Stufe über Separator (2) ausgespeiste Schwefelsäure (27) eine Konzentration zwischen 70 und 80% H₂SO₄ hat. Durch Vermischen der beiden Teilströme ergibt sich wieder eine Konzentration von ca. 78 bis 82% H₂SO₄ bei Eintritt in den Absäureeindampfer (7).

Eine bevorzugte Ausführung besteht darin, dass die gesamte 83- bis 86%ige Absäure (24) der Dinitrierung (3) in die Mononitrierung (1) eingeleitet wird und statt ca. 99%9ger Salpetersäure (25) eine 64- bis 70%ige Salpetersäure für die Mononitrierung eingesetzt wird.

Eine weitere Ausführung des erfindungsgemässen Verfahrens besteht darin, dass die ca. 90- bis 94%ige Schwefelsäure (29) in einer Hochkonzentrierungsstufe auf 94 bis 97% H₂SO₄ eingedampft wird und die hochkonzentrierte Säure bei der Dinitrierung (3) eingesetzt wird. Zur Vermeidung von Verstopfungen durch die geringen Mengen Dinitrotoluol, die bei der Hochkonzentrierung verdampfen, kann in das Brüdenkondensationssystem der Hochkonzentrierung ebenfalls, gegebenenfalls periodisch, MNT eingespritzt werden.

Alternativ zum beschriebenen Horizontalverdampfer für die Absäureeindampfung auf ca. 88 bis 94% H₂SO₄ können andere Unterdruckverdampfer eingesetzt werden.

Bei grösseren Anlagen kann es vorteilhaft sein, die Eindampfung der Absäure auf 88 bis 94% H₂SO₄ in mehreren hintereinandergeschalteten Verdampfern durchzuführen, die bei verschiedenem Unterdruck betrieben werden.

Metallsalze, die mit der Salpetersäure oder durch Korrosion in die Schwefelsäure gelangen, können bei längerem Betrieb des Säurekreislaufs aus der konzentrierten Säure beim Abkühlen im Wärmetauscher (6) auskristallisieren und müssen durch periodisches Spülen mit Wasser oder verdünnter Säure entfernt werden.

Die Vorteile des erfindungsgemässen Verfahrens sollen durch folgende Beispiele demonstriert werden, ohne dass dadurch eine Einschränkung des Umfanges der Erfindung geschieht. Die Eindampfung der Absäure erfolgt entsprechend Fig. 1, auf die bei den Beispielen Bezug genommen wird.

*Beispiel 1*  (Vergleichsbeispiel)
Die Absäure (27) aus der Mononitrierung (1) hatte folgende Zusammensetzung:

76,0% H₂SO₄
1,5% Nitrosylschwefelsäure
0,4% DNT
0,15% MNT
0,03% HNO₃

3,4 t/h dieser Absäure wurden im Wärmetauscher (6), zwei hintereinandergeschalteten Röhrenwärmetauschern aus Glas, auf 100°C vorgewärmt und in den Horizontalverdampfer (7) eingespeist. Das Tantalrohrbündel des Verdampfers wurde mit Sattdampf (37) von 195°C beheizt. Der Dampfverbrauch betrug 1,2 t/h. Die Wasserverdampfung aus der Absäure erfolgte bei einem Druck von 45 mbar. Es wurde 2,777 t/h konzentrierte Säure (35) mit 182°C ausgespeist, die im Wärmetauscher auf 40°C gekühlt wurden, bevor sie in die Dinitrierungsstufe (2) eingespeist wurden. Die konzentrierte Säure (35) hatte folgende Zusammensetzung:

92,0% H₂SO₄
1,4% Nitrosylschwefelsäure
0,007% DNT
MNT und HNO₃ waren nicht nachweisbar.

Die überhitzten Brüden (41) wurden durch Eindüsen von 100 l/h der wässrigen Phase des Brüdenkondensats (40) auf ca. 40°C abgekühlt, bevor sie in den Brüdenkondensator (8), einen wassergekühlten Rohrbündelwärmetauscher, gelangten. Das Brüdenkondensat (44) floss mit einer Temperatur von 25 bis 30°C in die Vorlage (9) ab. Der Unterdruck von 45 mbar wurde mittels einer Wasserringpumpe aufrecht erhalten, mit der die nichtkondensierbaren Gase (46) abgezogen und einer Natronlaugewäsche zwecks Entfernung der nitrosen Gase zugeführt wurden.

Unmittelbar nach Inbetriebnahme der Absäureeindampfung bildeten sich Beläge von festem DNT auf den Wandungen der gläsernen Brüdenleitung vom Verdampfer (7) zum Kondensator (8). Nach etwa 5 h kam die Verdampfung zum Erliegen, weil die Rohre des Brüdenkondensators (8) im oberen Bereich weitgehend durch festes DNT blockiert waren, so dass der erforderliche Unterdruck im Verdampfer nicht mehr aufrecht erhalten werden konnte. Auch die Brüdenkondensatleitung vom Kondensator (8) zur Vorlage (9) war an mehreren Stellen weitgehend durch festes DNT blockiert.

*Beispiel 2*

Die Verdampfung erfolgte analog zu Beispiel 1, jedoch wurde mit dem wässrigen Brüdenkondensat (40) 55 kg/h Roh-MNT (28) in die überhitzten Brüden eingedüst. Dadurch wurde die Abscheidung von festem DNT im Brüdenkondensationssystem vermieden. Die aus der Vorlage (9) abgezogene organische Phase des Brüdenkondensats (44) war flüssig. Sie enthielt die 4,3fache Menge MNT, bezogen auf DNT. Sie wurde zusammen mit dem Auslauf (26) der Mononitrierung (1) in denSeparator (2) eingespeist.

*Beispiel 3*

Im HOrizontalverdampfer (7) wurden 3 t/h Absäure (entsprechend Beispiel 1) bei einem Druck von 100 mbar auf 89,0% $H_2SO_4$ eingedampft. Die Wasserverdampfung betrug 422 kg/h. Die ausgespeiste Säure enthielt noch 0,02% DNT. Bei Temperaturen von 45°C am Kondensatoreintritt und 30°C am Kondensatoraustritt genügte die Zugabe von 30 kg/h Roh-MNT (28) zur eingespeisten Absäure, um die Abscheidung festen DNTs im Brüdenkondensationssystem zu vermeiden. In der organischen Phase des Brüdenkondensats (44) lag das MNT:DNT-Verhältnis bei 2,8:1.

**Patentansprüche**

1. Verfahren zur Herstellung von Dinitrotoluol durch zweistufige Umsetzung von Toluol mit Salpetersäure in Gegenwart von Schwefelsäure, wobei in der ersten Stufe Toluol zu Mononitrotoluol nitriert wird unter Verwendung von Absäure aus der zweiten Stufe, in der das Mononitrotoluol zu Dinitrotoluol nitriert wird unter Verwendung von aufkonzentrierter Absäure aus der ersten Stufe, dadurch gekennzeichnet, dass die Absäure in indirekt beheizten Eindampfern unter Vakuum aufkonzentriert wird und Mononitrotoluol in die überhitzten Brüden des Eindampfers gegeben wird.

2. Verfahren gemäss Anspruch 1, dadurch gekennzeichnet, dass das Mononitrotoluol in Form von mononitrotoluolhaltigen Gemischen, bevorzugt als Teil der organischen Phase, die nach der Toluolmononitrierung abgetrennt wird, zugegeben wird.

3. Verfahren gemäss einem der Ansprüche 1 oder 2, dadurch gekennzeichnet, dass in der organischen Phase des Kondensats der Brüden ein Verhältnis von Mononitrotoluol zu Dinitrotoluol von 2:1 bis 10:1, vorzugsweise 4:1 bis 7:1, vorliegt.

4. Verfahren gemäss einem der Ansprüche 1 bis 3, dadurch gekennzeichnet, dass Wasser oder bevorzugt ein Teil der wässrigen Phase des Brüdenkondensats in die überhitzten Brüden zugedüst wird.

5. Verfahren gemäss einem der Ansprüche 1 bis 4, dadurch gekennzeichnet, dass die Absäure auf einen Schwefelsäuregehalt von 88 bis 94% $H_2SO_4$ eingedampft und bei der Dinitrotoluolherstellung und danach bei der Mononitrotoluolherstellung eingesetzt wird.

6. Verfahren gemäss einem der Ansprüche 1 bis 5, dadurch gekennzeichnet, dass die Eindampfung der Absäure in Horizontalverdampfern erfolgt.

7. Verfahren gemäss einem der Ansprüche 1 bis 5, dadurch gekennzeichnet, dass die Eindampfung der Absäure in Fallfilm- oder Umlaufverdampfern erfolgt.

8. Verfahren gemäss einem der Ansprüche 1 bis 7, dadurch gekennzeichnet, dass ein Teil der nach der Dinitrotoluolherstellung abgetrennten Absäure bei der Mononitrotoluolherstellung eingesetzt wird und danach zusammen mit dem anderen Teil der Absäure aus der Dinitrotoluolherstellung auf einen Schwefelsäuregehalt von 88 bis 94% eingedampft wird.

9. Verfahren gemäss Ansprüchen 1 bis 7, dadurch gekennzeichnet, dass die 88- bis 94%ige Schwefelsäure bei der Dinitrotoluolherstellung zusammen mit 98- bis 100%iger $HNO_3$ und anschliessend bei der Mononitrotoluolherstellung zusammen mit 63- bis 70%iger $HNO_3$ eingesetzt wird.

10. Verfahren gemäss einem der Ansprüche 1 bis 9, dadurch gekennzeichnet, dass die Absäure auf einen Schwefelsäuregehalt von 88 bis 94% eingedampft wird und anschliessend in einer Hochkonzentrierungsstufe auf 94 bis 97% $H_2SO_4$ eingedampft wird, bevor sie wieder bei der Dinitrotoluolherstellung eingesetzt wird.

**Claims**

1. Process for the production of dinitrotoluene by a two-stage reaction of toluene with nitric acid in the presence of sulphuric acid, wherein toluene is nitrated to mononitrotoluene in the first stage using spent acid from the second stage, in which the mononitrotoluene ist nitrated to dinitrotoluene using concentrated spent acid from the first stage, characterised in that the spent acid is concentrated in vacuo in indirectly heated evaporators and mononitrotoluene is fed into the superheated vapours of the evaporator.

2. Process according to Claim 1, characterised in that the mononitrotoluene is added in the form of mononitrotoluene-containing mixtures, preferably as a part of the organic phase which is separated off after the mononitration of the toluene.

3. Process according to one of Claims 1 or 2, characterised in that a ratio mononitrotoluene to dinitrotoluene of 2:1 to 10:1, preferably 4:1 to 7:1, is present in the organic phase of the condensate of the vapours.

4. Process according to one of Claims 1 to 3, characterised in that water or preferably a portion of the aqueous phase of the vapour condensate is injected into the superheated vapours.

5. Process according to one of Claims 1 to 4, characterised in that the spent acid is evaporated to a sulphuric acid content of 88 to 94% of $H_2SO_4$ and used in the production of dinitrotoluene and then in the production of mononitrotoluene.

6. Process according to one of Claims 1 to 5, characterised in that the spent acid is evaporated in horizontal evaporators.

7. Process according to one of Claims 1 to 5, characterised in that the spent acid is evaporated in falling-film or circulation evaporators.

8. Process according to one of Claims 1 to 7, characterised in that a portion of the spent acid separated off after the production of dinitrotoluene is used in the production of mononitrotoluene and then evaporated, together with the other portion of the spent acid from the production of dinitrotoluene, to a sulphuric acid content of 88 to 94%.

9. Process according to Claims 1 to 7, characterised in that the 88 to 94% strength sulphuric acid is used together with 98 to 100% strength $HNO_3$ in the production of dinitrotoluene and then together with 63 to 70% strength $HNO_3$ in the production of mononitrotoluene.

10. Process according to one of Claims 1 to 9, characterised in that the spent acid is evaporated to a sulphuric acid content of 88 to 94% and is then evaporated to 94 to 97% of $H_2SO_4$ in a high concentration stage before it is reused in the production of dinitrotoluene.

## Revendications

1. Procédé de production de dinitrotoluène par réaction en deux étapes de toluène avec l'acide nitrique en présence d'acide sulfurique, de façon telle que dans la première étape, du toluène est nitré en mononitrotoluène avec utilisation d'acide résiduaire de la seconde étape, dans laquelle le mononitrotoluène est nitré en dinitrotoluène avec utilisation d'acide résiduaire concentré venant de la première étape, caractérisé en ce que l'acide résiduaire est concentré sous vide dans des évaporateurs à chauffage indirect et le mononitrotoluène est envoyé dans les vapeurs surchauffées de l'évaporateur.

2. Procédé suivant la revendication 1, caractérisé en ce que le mononitrotoluène est ajouté sous la forme de mélanges contenant du mononitrotoluène, de préférence comme partie de la phase organique qui est séparée après la mononitration du toluène.

3. Procédé suivant les revendications 1 ou 2, caractérisé en ce qu'un rapport du mononitrotoluène au dinitrotoluène de 2:1 à 10:1, de préférence de 4:1 à 7:1, existe dans la phase organique du condensat des vapeurs.

4. Procédé suivant l'une des revendications 1 à 3, caractérisé en ce que de l'eau ou de préférence une partie de la phase aqueuse du condensat des vapeurs est injectée das les vapeurs surchauffées.

5. Procédé suivant l'une des revendications 1 à 4, caractérisé en ce que l'acide résiduaire est concentré par évaporation à une teneur en acide sulfurique de 88 à 94% de $H_2SO_4$ et est utilisé dans la production de dinitrotoluène, puis dans la production de mononitrotoluène.

6. Procédé suivant l'une des revendications 1 à 5, caractérisé en ce que la concentration par évaporation de l'acide résiduaire est effectuée dans des évaporateurs horizontaux.

7. Procédé suivant l'une des revendications 1 à 5, caractérisé en ce que la concentration par évaporation de l'acide résiduaire est effectuée dans des évaporateurs à film tombant ou à circulation.

8. Procédé suivant l'une des revendications 1 à 7, caractérisé en ce qu'une partie de l'acide résiduaire séparé après la production du dinitrotoluène est utilisée dans la production de mononitrotoluène puis est concentrée par évaporation à une teneur en acide sulfurique de 88 à 94% conjointement avec l'autre partie de l'acide résiduaire venant de la production de dinitrotoluène.

9. Procédé suivant les revendications 1 à 7, caractérisé en ce que l'acide sulfurique à 88 à 94% est utilisé dans la production de dinitrotoluène conjointement avec de l'acide nitrique à 98 à 100%, puis dans la production de mononitrotoluène conjointement avec de l'acide nitrique à 63 à 70%.

10. Procédé suivant l'une des revendications 1 à 9, caractérisé en ce que l'acide résiduaire est concentré par évaporation à une teneur en acide sulfurique de 88 à 94% puis concentré par évaporation à 94 à 97% dans une étape de haute concentration, avant d'être réutilisé dans la production de dinitrotoluène.

FIG. 1